# EUROPEAN PATENT SPECIFICATION

(11) **EP 2 964 929 B1**
(45) Date of publication and mention of the grant of the patent: **12.02.2020**
(21) Application number: 14760264.3
(22) Date of filing: 06.03.2014
(51) Int. Cl.: F01D 25/20, G01N 33/28

(54) **OIL SYSTEM DEBRIS MONITOR SYSTEM FOR A GAS TURBINE ENGINE**
ABLAGERUNGSÜBERWACHUNGSSYSTEM FÜR ÖLSYSTEM BEI EINEM GASTURBINENMOTOR
SYSTÈME DE SURVEILLANCE DE DÉBRIS DE SYSTÈME D'HUILE DESTINÉ À UN MOTEUR À TURBINE À GAZ

(30) Priority: 06.03.2013 US 201361773618 P
(43) Date of publication of application: 13.01.2016
(73) Proprietor: United Technologies Corporation, Farmington, CT 06032 (US)
(72) Inventor: PARNIN, Francis, Suffield, Connecticut 06078 (US); MCCUNE, Michael E., Colchester, Connecticut 06415 (US)
(74) Representative: Dehns
(86) International application number: PCT/US2014/021299
(87) International publication number: WO 2014/138432

(56) References cited:
- EP-A2- 0 819 938
- EP-A2- 2 246 529
- US-A- 4 677 425
- US-A- 4 888 948
- US-A1- 2009 014 245
- US-A1- 2010 031 659
- US-A1- 2010 066 354
- US-A1- 2010 313 639
- Detlef Aigner: "Gleichung zur Berechnung der hydraulischen Verluste der Rohrvereinigung - kalibriert mit Ergebnissen numerischer und physikalischer Modelle", , 15 January 2008 (2008-01-15), XP055299633, Retrieved from the Internet: URL:http://rcswww.urz.tu-dresden.de/~daign er/pdf/vereinig.pdf [retrieved on 2016-09-02]
- ANDRÉ GARDEL ET AL: "Les pertes de charge dans les branchements en Té des conduites de section circulaire", SOCIÉTÉ ANONYME DU BULLETIN TECHNIQUE DE LA SUISSE ROMANDE, vol. 96, no. 25, 12 December 1970 (1970-12-12), pages 363-391, XP055299648, DOI: 10.5169/seals-70882

## Description

### BACKGROUND

The present disclosure relates to a gas turbine engine and, more particularly, to a debris monitor sensor system arrangement therefor.

Aircraft gas turbine engines include an oil system to supply oil to various components such as a fan drive gear system. These engine components may have moving parts that may shed small debris particles, or particulates, to the lubricating liquid. A debris monitor sensor system is often used to monitor the shed particulates. By monitoring particulates, maintenance personnel gain valuable information about component wearing for maintenance decisions. A filter is often used to remove the particulates from the lubricant before lubrication of engine components.

US 4,667,425 discloses a prior art oil debris monitor sensor system in accordance with the preamble of claim 1, and a prior art method according to the preamble of claim 12.

US 2010/0066354 A1 discloses a prior art metallic debris detection sensor.

US 5,973,503 discloses prior art process and measurement systems for measuring physical quantities of poorly conductive and nonconductive fluids.

### SUMMARY

According to a first aspect of the present invention, there is provided an oil debris monitor sensor system as set forth in claim 1.

According to a further aspect of the present invention, there is provided a method as set forth in claim 12.

Further possible embodiments of the invention are provided, as set forth in dependent claims 2 to 11 and 13 to 14.

### BRIEF DESCRIPTION OF THE DRAWINGS

Various features will become apparent to those skilled in the art from the following detailed description of the disclosed non-limiting embodiment. The drawings that accompany the detailed description can be briefly described as follows:
Figure 1 is a schematic cross-sectional view of a geared architecture gas turbine engine;
Figure 2 is a schematic view of an oil system for the geared architecture gas turbine engine;
Figure 3 is a schematic view of a debris monitor sensor system according to one disclose non-limiting embodiment;
Figure 4 is a schematic view of a debris monitor sensor system according to another disclose non-limiting embodiment; and
Figure 5 is a schematic view of a debris monitor sensor system according to another disclose non-limiting embodiment.

### DETAILED DESCRIPTION

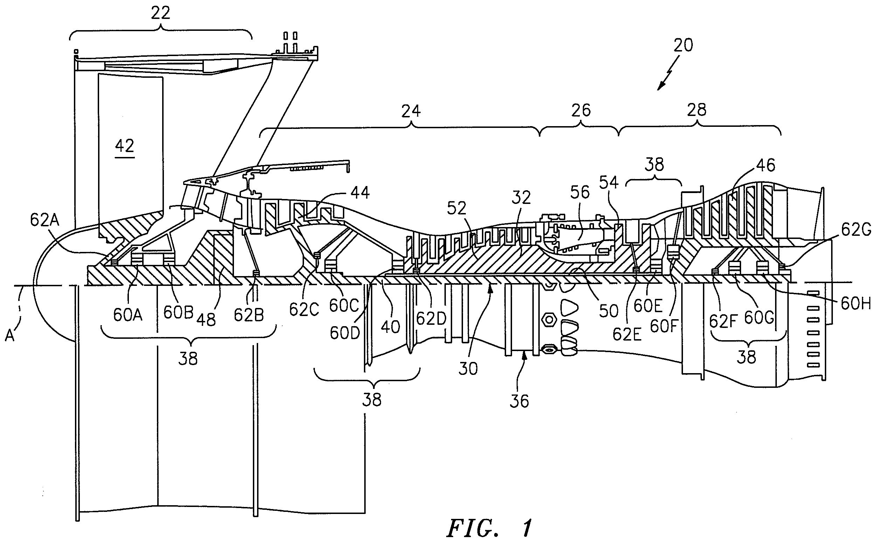

Figure 1 schematically illustrates a gas turbine engine 20. The gas turbine engine 20 is disclosed herein as a two-spool turbofan that generally incorporates a fan section 22, a compressor section 24, a combustor section 26 and a turbine section 28. Alternative engines architectures such as a low-bypass turbofan may include an augmentor section (not shown) among other systems or features. Although schematically illustrated as a turbofan in the disclosed non-limiting embodiment, it should be understood that the concepts described herein are not limited to use with turbofans as the teachings may be applied to other types of turbine engines to include but not limited to a three-spool (plus fan) engine wherein an intermediate spool includes an intermediate pressure compressor (IPC) between a low pressure compressor and a high pressure compressor with an intermediate pressure turbine (IPT) between a high pressure turbine and a low pressure turbine as well as other engine architectures such as turbojets, turboshafts, open rotors and industrial gas turbines.

The fan section 22 drives air along a bypass flowpath and a core flowpath while the compressor section 24 drives air along the core flowpath for compression and communication into the combustor section 26 then expansion through the turbine section 28. The engine 20 generally includes a low spool 30 and a high spool 32 mounted for rotation about an engine central longitudinal axis A relative to an engine case assembly 36 via several bearing compartments 38. The low spool 30 generally includes an inner shaft 40 that interconnects a fan 42, a low pressure compressor 44 ("LPC") and a low pressure turbine 46 ("LPT"). The inner shaft 40 drives the fan 42 through a geared architecture 48 to drive the fan 42 at a lower speed than the low spool 30.

The high spool 32 includes an outer shaft 50 that interconnects a high pressure compressor 52 ("HPC") and high pressure turbine 54 ("HPT"). A combustor 56 is arranged between the HPC 52 and the HPT 54. The inner shaft 40 and the outer shaft 50 are concentric and rotate about the engine central longitudinal axis A that is collinear with their longitudinal axes.

Core airflow is compressed by the LPC 44 then the HPC 52, mixed with the fuel and burned in the combustor section 56, then expanded over the HPT 54 and the LPT 46. The HPT 54 and the LPT 46 drive the respective high spool 32 and low spool 30 in response to the expansion.

In one non-limiting example, the gas turbine engine 20 is a high-bypass geared architecture engine in which the bypass ratio is greater than about six (6:1). The geared architecture 48 can include an epicyclic gear train, such as a planetary gear system, star gear system or other gear system. The example epicyclic gear train has a gear reduction ratio of greater than about 2.3, and in another example is greater than about 2.5 with a gear system efficiency greater than approximately 98%. The geared turbofan enables operation of the low spool 30 at higher speeds which can increase the operational efficiency of the low pressure compressor 44 and low pressure turbine 46 and render increased pressure in a fewer number of stages.

A pressure ratio associated with the LPT 46 is pressure measured prior to the inlet of the LPT 46 as related to the pressure at the outlet of the LPT 46 prior to an exhaust nozzle of the gas turbine engine 20. In one non-limiting embodiment, the bypass ratio of the gas turbine engine 20 is greater than about ten (10:1), the fan diameter is significantly larger than that of the low pressure compressor 44, and the low pressure turbine 46 has a pressure ratio that is greater than about five (5:1). It should be understood, however, that the above parameters are only exemplary of one embodiment of a geared architecture engine and that the present disclosure is applicable to other gas turbine engines including direct drive turbofans.

A significant amount of thrust is provided by the bypass flow due to the high bypass ratio. The fan section 22 of the gas turbine engine 20 is designed for a particular flight condition - typically cruise at about 0.8 Mach and about 35,000 feet (10,668 m). This flight condition, with the gas turbine engine 20 at its best fuel consumption, is also known as bucket cruise Thrust Specific Fuel Consumption (TSFC). TSFC is an industry standard parameter of fuel consumption per unit of thrust.

Fan Pressure Ratio is the pressure ratio across a blade of the fan section 22 without the use of a Fan Exit Guide Vane system. The low Fan Pressure Ratio according to one non-limiting embodiment of the example gas turbine engine 20 is less than 1.45. Low Corrected Fan Tip Speed is the actual fan tip speed divided by an industry standard temperature correction of ("Tram" / 518.7)^{0.5} (where °R = K x 9/5) in which "Tram" represents the ambient temperature in degrees Rankine. The Low Corrected Fan Tip Speed according to one non-limiting embodiment of the example gas turbine engine 20 is less than about 1150 fps (351 m/s).

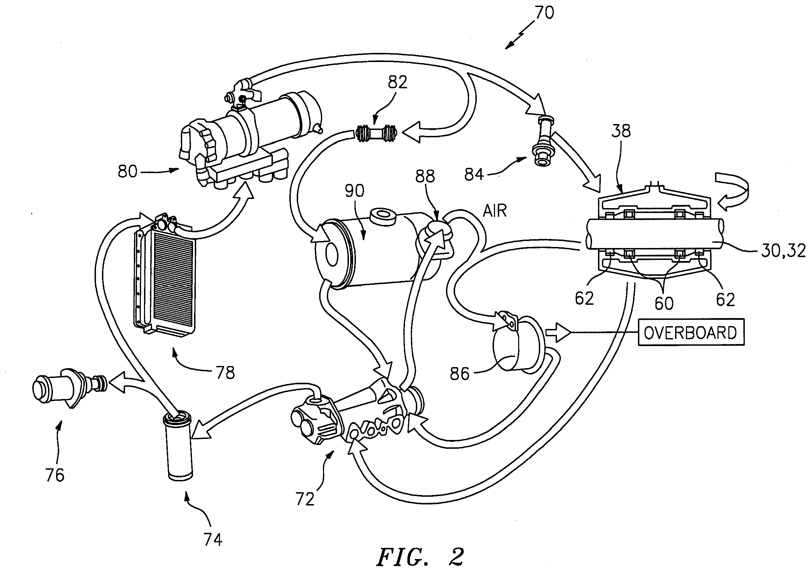

As illustrated in Figure 2, each of the bearing compartments 38 includes one or more bearings 60A-60H and one or more seals 62A-62G. Various types of seals 62A-62G may be used. The bearings 60A-60H and seals 62A-62G respectively support and interface with the shafts 40, 50 of the respective low spool 30 and high spool 32. The seals 62A-62G operate to seal a "wet" zone from a "dry" zone. For ease of reference, regions or volumes that contain oil may be referred to as a "wet" zone and an oil-free region may be referred to as a "dry" zone. So, for example, the interior of each bearing compartment 38 and the geared architecture 48 may be referred to as a wet zone that ultimately communicates with an oil sump (not shown) of an oil system (Figure 2) while the region external thereto may be referred to as a dry zone. That is, the bearings 60A-60H support the low spool 30 and the high spool 32 and the seals 62A-62G separates the "wet" zone from the "dry" zone to define the boundaries of each bearing compartment 38.

With reference to Figure 2, an oil system 70 provides oil under pressure to lubricate and cool moving components of the engine 20, such as, for example, the geared architecture 48 and the bearings 60. The oil system 70 generally includes a pump 72, a main filter 74, a pressure relief valve 76, an air-oil cooler 78, a fuel-oil cooler 80, an oil pressure trim orifice 82, an oil strainer 84, a deoiler 86, a deareator 88 and a tank 90. The oil system 70 is but a schematic illustration and other additional or alternative components and subsystems may be included in the oil system 70.

In operation, the pump 72 communicates oil to the main filter 74, and then filtered oil proceeds to the air-oil cooler 78 and/or the fuel-oil cooler 80 to be cooled. Should the main filter 74 become clogged, the pressure relief valve 76 indicates the clog. The oil pressure trim orifice 82 then regulates the oil flow and returns any oil overage to the oil tank 90. The oil strainer 84 strains any debris before the oil is communicated to, for example, the geared architecture 48 and the bearings 60.

Oil is then scavenged from the bearing compartment 38 and geared architecture 48 by the pump 72 through the deaerator 88 for return to the tank 90. Air is separated from the scavenge oil from the pump 72 by the deaerator 88 at the tank 90. Vent air from the for example the geared architecture 48 and the bearings 60A-60Hand air from the deaerator 88 is communicated directly to the deoiler 86 where the air is removed from the oil and rejected overboard. The oil cycle is then repeated.

Geared architecture engines are architected to minimize oil flow, heat rejection, air entrainment in the oil in regular operation. Geared architecture engines, however, typically have an oil flow circulation that may be approximately twice that of a traditional direct drive turbofan, e.g., 45 gallons per minute versus 25 gallons per minute from a 35 quart oil tank vs. a 28 quart system.

Geared architecture engines, as all gas turbine engines, may shed small debris particles, or particulates, to the lubricating liquid. Debris monitoring sensor system 100 monitors the shed particulates. The minimum particle size detection capability of debris monitoring sensors in lubrication systems is directly related to size of the sensor flow passage. The smaller the sensor flow passage, the smaller the particle size the sensor can detect. The size of the flow passage also affects the fluid pressure drop of the sensor, the smaller the passage the larger the pressure drop. In high oil flow rate systems such as an engine equipped with a geared architecture 48, pressure drop associated with the sensor passage size required to detect wear particles may become prohibitively high when driving the full engine lubricant flow through the sensor, as is the typical practice.

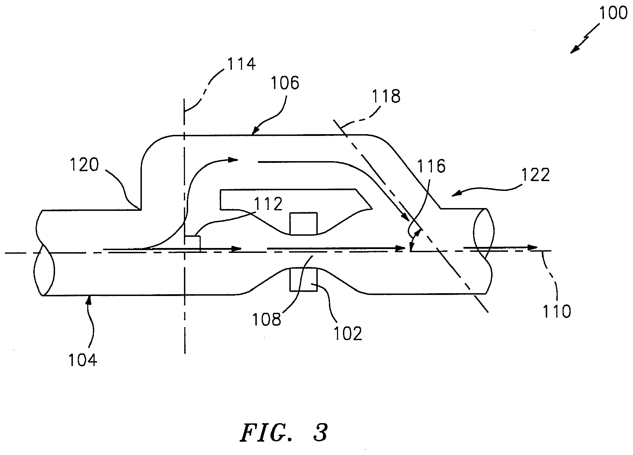

With reference to Figure 3, the debris monitor sensor system 100 generally includes an oil debris monitor 102, a first passage 104 and a second passage 106. The debris monitor sensor system 100 may be located anywhere within the oil system 70, such as, for example, downstream of the pump 72, upstream of the deareator 88 or other location. Furthermore, multiple debris monitor sensor systems 100 may be utilized.

The oil debris monitor 102 such as that manufactured by Gastops Ltd, Ottawa, Ontario, Canada is located in or around a sensor passage section 108 in the first passage 104. In one example, the oil debris monitor 102 may identify particles greater than about 150 microns (0.006 inches). The sensor passage section 108 is in line with the first passage 104 along a first axis 110. In one non-limiting example, the sensor passage section 108 is about 0.6" (15mm) in diameter and the first passage 104 is about 1" (25mm) in diameter. In another non-limiting example, the sensor passage section 108 is about 1" (25mm) in diameter and the first passage 104 is about 1.75" (44.5mm) in diameter.

The first passage 104 is displaced from the second passage 106 and may be generally parallel thereto. In one disclosed non-limiting embodiment, the first passage 104 and the second passage 106 are linearly offset, the first passage 104 defines a first gravitational potential and the second passage 106 having a second gravitation potential, the first gravitational potential different than the second gravitation potential. That is, the different gravitational potential facilitates debris separation.

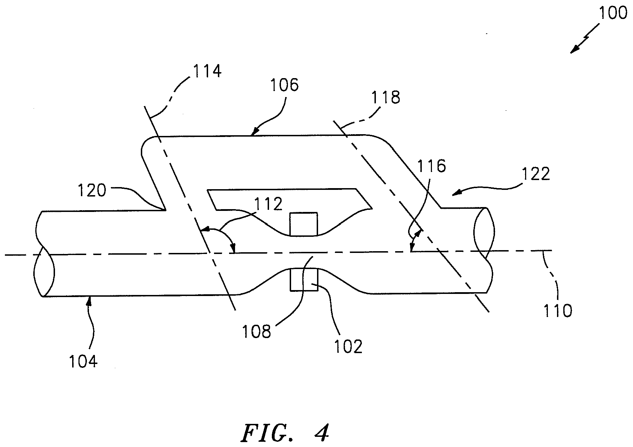

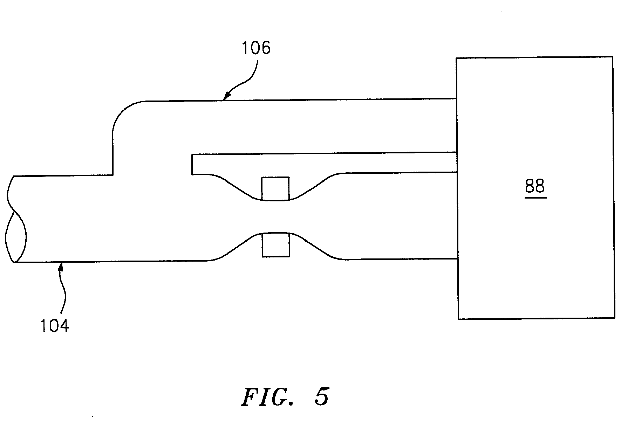

The first passage 104 is below the second passage 106. The second passage 106 splits from the first passage 104 at a first angle 112 defined between the first axis 110 and a second axis 114. The second passage 106 then rejoins the first passage 104 at a second angle 116 defined between a third axis 118 and the first axis 110. Intersections 120, 122 between the second passage 106 and the first passage 104 may be smooth or streamlined to facilitate oil flow. In one disclosed non-limiting embodiment, the first angle 112 is about 90 degrees and the second angle 116 is between 20-45 degrees. In another disclosed non-limiting embodiment, the first angle 112 is greater than 90 degrees and the second angle 116 is between 20-45 degrees (Figure 4). In yet another disclosed non-limiting embodiment, the second passage 106 and the first passage 104 may continue separately to an oil system component such as the deaerator 88 (Figure 5).

The weight and momentum of the wear particles in the lubricant results in a significant amount of wear particles continuing to flow through a flowpath with the sensor passage section 108. The debris monitor sensor system 100 bypasses excess lubricant flow around the sensor passage section 108 through the second passage 106 to minimize pressure drop and also minimizes the proportion of wear particles that bypass the oil debris monitor 102.

It should be understood that like reference numerals identify corresponding or similar elements throughout the several drawings.

## Claims

1. An oil debris monitor sensor system (100) comprising:
a flow path of an oil system (70) having a flow direction, the flow path comprising:
a first passage (104) having a first axis (110) and including a sensor passage section (108);
a second passage (106) that splits from said first passage (104); and
an oil debris monitor (102) located in or around said sensor passage section (108),
**characterised in that**:
the first passage (104) is disposed substantially along the flow direction of the flow path oil system; and
said second passage (106) splits from said first passage (104) at a first angle (112) with respect to the first axis (110) of said first passage (104) such that said first passage (104) is below the second passage (106).

2. The system (100) as recited in claim 1, wherein said first angle (112) is about 90 degrees with respect to said first axis (110).

3. The system (100) as recited in claim 2, wherein said first angle (112) is greater than 90 degrees with respect to said first axis (110).

4. The system (100) as recited in claim 2, wherein said first angle (112) is greater than 45 degrees with respect to said first axis (110).

5. The system (100) as recited in any preceding claim, wherein said second passage (106) rejoins said first passage (104) downstream of said sensor passage section (108).

6. The system (100) as recited in claim 5, wherein said second passage (106) rejoins said first passage (108) downstream of said sensor passage section (108) at a second angle (116).

7. The system (100) as recited in claim 6, wherein said second angle (116) is about 20-45 degrees with respect to said first axis (110).

8. The system (100) as recited in any preceding claim, wherein said second passage (106) defines a diameter generally equal to said first passage (104).

9. The system (100) as recited in any of claims 1 to 7, wherein said second passage (106) defines a diameter smaller than said first passage (104).

10. The system (100) as recited in any preceding claim, wherein said first passage (104) and said second passage (106) communicate separately with an oil system component.

11. The system (100) as recited in claim 10, wherein said oil system component is a deaerator (88).

12. A method of configuring an oil debris monitoring system for a gas turbine engine (20), comprising:
in a flow path of an oil system (70) having a flow direction, splitting and rejoining a second passage (106) of an oil system (70) from a first passage (104) of the oil system (70), around a sensor passage section (108) which is in the first passage (104), to direct debris through the sensor passage section (108);
**characterised in that**:
the first passage (104) is below the second passage (106);
said second passage (106) splits from said first passage (104) at a first angle (112) with respect to
an axis (110) of said first passage (104), said first passage (104) disposed substantially linearly said flow direction of said flow path.

13. The method as recited in claim 12, further comprising:
splitting the second passage (106) from the first passage (104) at a first angle (112) of about 90 degrees.

14. The method as recited in claim 12, further comprising:
splitting the second passage (106) from the first passage (104) at a first angle (112) greater than 90 degrees.

## Patentansprüche

1. Ölablagerungsüberwachungssensorsystem (100), umfassend:
einen Strömungsweg eines Ölsystems (70), das eine Strömungsrichtung aufweist, wobei der Strömungsweg Folgendes umfasst:
einen ersten Durchlass (104), der eine erste Achse (110) aufweist und einen Sensordurchlassabschnitt (108) beinhaltet;
einen zweiten Durchlass (106), der sich von dem ersten Durchlass (104) trennt; und
einen Ölablagerungsmonitor (102), der sich in dem oder um den Sensordurchlassabschnitt (108) befindet,
**dadurch gekennzeichnet, dass**:
der erste Durchlass (104) im Wesentlichen entlang der Strömungsrichtung des Strömungswegölsystems angeordnet ist; und
sich der zweite Durchlass (106) in einem ersten Winkel (112) in Bezug auf die erste Achse (110) des ersten Durchlasses (104) von dem ersten Durchlass (104) trennt, sodass sich der erste Durchlass (104) unter dem zweiten Durchlass (106) befindet.

2. System (100) nach Anspruch 1, wobei der erste Winkel (112) etwa 90 Grad in Bezug auf die erste Achse (110) beträgt.

3. System (100) nach Anspruch 2, wobei der erste Winkel (112) in Bezug auf die erste Achse (110) größer als 90 Grad ist.

4. System (100) nach Anspruch 2, wobei der erste Winkel (112) in Bezug auf die erste Achse (110) größer als 45 Grad ist.

5. System (100) nach einem vorhergehenden Anspruch, wobei sich der zweite Durchlass (106) stromabwärts des Sensordurchlassabschnittes (108) wieder mit dem ersten Durchlass (104) verbindet.

6. System (100) nach Anspruch 5, wobei sich der zweite Durchlass (106) stromabwärts des Sensordurchlassabschnittes (108) in einem zweiten Winkel (116) wieder mit dem ersten Durchlass (108) verbindet.

7. System (100) nach Anspruch 6, wobei der zweite Winkel (116) etwa 20-45 Grad in Bezug auf die erste Achse (110) beträgt.

8. System (100) nach einem vorhergehenden Anspruch, wobei der zweite Durchlass (106) einen Durchmesser definiert, der im Allgemeinen gleich dem ersten Durchlass (104) ist.

9. System (100) nach einem der Ansprüche 1 bis 7, wobei der zweite Durchlass (106) einen Durchmesser definiert, der kleiner als der erste Durchlass (104) ist.

10. System (100) nach einem vorhergehenden Anspruch, wobei der erste Durchlass (104) und der zweite Durchlass (106) separat mit einer Ölsystemkomponente kommunizieren.

11. System (100) nach Anspruch 10, wobei die Ölsystemkomponente ein Entlüfter (88) ist.

12. Verfahren zum Konfigurieren eines Ölablagerungsüberwachungssystems für einen Gasturbinenmotor (20), umfassend:
in einem Strömungsweg eines Ölsystems (70), das eine Strömungsrichtung aufweist, Trennen und Wiederverbinden eines zweiten Durchlasses (106) eines Ölsystems (70) von einem ersten Durchlass (104) des Ölsystems (70), um einen Sensordurchlassabschnitt (108), der sich in dem ersten Durchlass (104) befindet, um Ablagerungen durch den Sensordurchlassabschnitt (108) zu leiten;
**dadurch gekennzeichnet, dass**:
sich der erste Durchlass (104) unter dem zweiten Durchlass (106) befindet;
sich der zweite Durchlass (106) in einem ersten Winkel (112) in Bezug auf eine Achse (110) des ersten Durchlasses (104) von dem ersten Durchlass (104) trennt, wobei der erste Durchlass (104) im Wesentlichen linear in der Strömungsrichtung des Strömungsweges angeordnet ist.

13. Verfahren nach Anspruch 12, ferner umfassend:
Trennen des zweiten Durchlasses (106) von dem ersten Durchlass (104) in einem ersten Winkel (112) von etwa 90 Grad.

14. Verfahren nach Anspruch 12, ferner umfassend:
Trennen des zweiten Durchlasses (106) von dem ersten Durchlass (104) in einem ersten Winkel (112), der größer als 90 Grad ist.

## Revendications

1. Système de capteur de surveillance de débris d'huile (100) comprenant :
un trajet d'écoulement d'un système d'huile (70) ayant une direction d'écoulement, le trajet d'écoulement comprenant :
un premier passage (104) ayant un premier axe (110) et comportant une section de passage de capteur (108) ;
un second passage (106) qui se sépare dudit premier passage (104) ; et
un dispositif de surveillance de débris d'huile (102) situé dans ou autour de ladite section de passage de capteur (108), **caractérisé en ce que** :
le premier passage (104) est disposé sensiblement le long de la direction d'écoulement du système d'huile à trajet d'écoulement ; et
ledit second passage (106) se sépare dudit premier passage (104) selon un premier angle (112) par rapport au premier axe (110) dudit premier passage (104) de sorte que ledit premier passage (104) se situe en dessous du second passage (106).

2. Système (100) selon la revendication 1, dans lequel ledit premier angle (112) est d'environ 90 degrés par rapport audit premier axe (110).

3. Système (100) selon la revendication 2, dans lequel ledit premier angle (112) est supérieur à 90 degrés par rapport audit premier axe (110).

4. Système (100) selon la revendication 2, dans lequel ledit premier angle (112) est supérieur à 45 degrés par rapport audit premier axe (110).

5. Système (100) selon une quelconque revendication précédente, dans lequel ledit second passage (106) rejoint ledit premier passage (104) en aval de ladite section de passage de capteur (108) .

6. Système (100) selon la revendication 5, dans lequel ledit second passage (106) rejoint ledit premier passage (108) en aval de ladite section de passage de capteur (108) selon un second angle (116) .

7. Système (100) selon la revendication 6, dans lequel ledit second angle (116) est d'environ 20 à 45 degrés par rapport audit premier axe (110).

8. Système (100) selon une quelconque revendication précédente, dans lequel ledit second passage (106) définit un diamètre généralement égal audit premier passage (104).

9. Système (100) selon l'une quelconque des revendications 1 à 7, dans lequel ledit second passage (106) définit un diamètre inférieur à celui dudit premier passage (104).

10. Système (100) selon une quelconque revendication précédente, dans lequel ledit premier passage (104) et ledit second passage (106) communiquent séparément avec un composant de système d'huile.

11. Système (100) selon la revendication 10, dans lequel ledit composant de système d'huile est un désaérateur (88).

12. Procédé de configuration d'un système de surveillance de débris d'huile destiné à un moteur à turbine à gaz (20), comprenant :
dans un trajet d'écoulement d'un système d'huile (70) ayant une direction d'écoulement, la séparation et la jonction d'un second passage (106) d'un système d'huile (70) à partir d'un premier passage (104) du système d'huile (70), autour d'une section de passage de capteur (108) qui se trouve dans le premier passage (104), pour diriger les débris à travers la section de passage de capteur (108) ;
**caractérisé en ce que** :
le premier passage (104) se situe en dessous du second passage (106) ;
ledit second passage (106) se sépare dudit premier passage (104) selon un premier angle (112) par rapport à un axe (110) dudit premier passage (104), ledit premier passage (104) étant disposé de manière sensiblement linéaire par rapport à ladite direction d'écoulement dudit trajet d'écoulement.

13. Procédé selon la revendication 12, comprenant en outre :
la séparation du second passage (106) du premier passage (104) selon un premier angle (112) d'environ 90 degrés.

14. Procédé selon la revendication 12, comprenant en outre :
la séparation du second passage (106) du premier passage (104) selon un premier angle (112) supérieur à 90 degrés.
